# EUROPÄISCHE PATENTSCHRIFT

(11) **EP 1 480 625 B1**
(45) Veröffentlichungstag und Bekanntmachung des Hinweises auf die Patenterteilung: **06.08.2008**
(21) Anmeldenummer: 03743380.2
(22) Anmeldetag: 05.03.2003
(51) Int. Cl.: A61K 9/70

(54) **TRANSDERMALSYSTEM MIT FENTANYL**
TRANSDERMAL SYSTEM COMPRISING FENTANYL
SYSTEME TRANSDERMIQUE AU FENTANYLE

(30) Priorität: 06.03.2002 DE 10209724
(43) Veröffentlichungstag der Anmeldung: 01.12.2004
(73) Patentinhaber: HEXAL AG, 83607 Holzkirchen (DE)
(72) Erfinder: TISA-BOSTEDT, Katalin, 82049 Pullach (DE); FISCHER, Wilfried, 83620 Vagen (DE); LEICHS, Christian, 83714 Miesbach (DE)
(74) Vertreter: Boeters, Hans Dietrich
(86) Internationale Anmeldenummer: PCT/EP2003/002249
(87) Internationale Veröffentlichungsnummer: WO 2003/074035

(56) Entgegenhaltungen:
- EP-A- 1 170 004
- DE-A- 19 708 674
- DE-C- 19 654 468
- DATABASE WPI Section Ch, Week 200019 Derwent Publications Ltd., London, GB; Class A96, AN 2000-217930 XP002241646 & JP 2000 044476 A (TOKO YAKUHIN KOGYO KK) , 15. Februar 2000 (2000-02-15)

## Beschreibung

### 1. Stand der Technik

Die transdermale Anwendung von Fentanyl von Tumor- und postoperativen Schmerzen ist bekannt (siehe US 4,588,580). Dieses Patent beschreibt neben der allgemeinen Anwendbarkeit von Fentanyl und seiner Derivate auch Arzneiformen zur transdermalen Anwendung. Es handelt sich dabei um Reservoirsysteme, die einerseits ein Flüssigreservoir für den Wirkstoff mit einem Resorptionsförderer, andererseits ein festes Reservoir für den Wirkstoff enthalten.
Die Nachteile der Reservoirsysteme ganz allgemein und im Speziellen für Fentanyl als Betäubungsmittel sind folgende:
- teure und aufwendige Herstellung
- leichte missbräuchliche Verwendung durch Wiederverwendung des flüssigen Reservoirinhaltes
- Abgabekontrollelement kann durch leichte mechanische Einwirkung zerstört werden
- weiterhin kann man keine Dosisanpassung vornehmen durch einfache Variation der Fläche eines solchen Systems, da die Membran zerstört wird
- hohe Gefahr der Hautirritation durch Verwendung flüssiger Resorptionsförderer
- flüssige Lösemittel führen aus Erfahrung zu einer verminderten Klebkraft des druckempfindlichen Haftklebers über die Lagerzeit

US 4,806,341 beschreibt mikrodispergierte Analgetika wie Fentanyl in einer nichtklebenden Polymerscheibe. In dieser Polymerscheibe ist die Wirkstofflösung in einem hydrophilen Lösemittel wie Propylenglykol in Form von feinen Tröpfchen in einem hydrophoben Polymer dispergiert. Die Polymerscheibe wird mit einem druckempfindlichen Haftkleber, der auf dieser aufgebracht ist, auf der Haut fixiert. Nachteile sind:
- komplizierte Herstellung
- flüssige Lösemittel führen aus Erfahrung zu einer verminderten Klebkraft des druckempfindlichen Haftklebers über die Lagerzeit
- dickes System und dadurch nicht hautanschmiegsam und variabler Kontakt, dies führt zu unzuverlässigen Blutspiegeln
- Hautirritationen durch flüssige Lösemittel/Dispersionsmittel

US 5,091,186 beschreibt biphasische transdermale Systeme, auch für Fentanyl, bei denen auf eine Wirkstoffreisetzung von 10 bis 14 Stunden eine wirkstoffabgabefreie Phase folgt.
Nachteile dieser Formulierung für Fentanyl sind folgende:
- nicht kontinuierliche Freigabe
- dieses System erscheint eher geeignet für Substanzen wie Nitroglycerin, die eine wirkstoffreie Abgabephase zur Vermeidung von Toleranzentwicklung erfordern.

US 5,816,939 beschreibt ein transdermales System für die Verabreichung von Fentanyl in Matrices aus druckempfindlichen Haftklebern, welche >5 % Fentanyl enthalten und als Resorptionsförderer Propylenglykolmonolaurat (PGML) verwenden. Der Nachteil dieses System ist, dass ca. 75 % der Fentanyl-Gesamtdosis am ersten Tag abgegeben werden, so daß das System nicht für eine Mehrtagesbehandlung geeignet ist.

US 5,006,342 beschreibt ein mehrschichtiges Laminat für die transdermale Anwendung für Fentanyl, bei dem eine wirkstoffhaltige Schicht zwischen zwei elastischen strukturgebenden Schichten eingebettet ist. Die wirkstoffhaltige Polymerschicht kann löslichkeits- oder hautpermeationsfördernde Substanzen wie Polyethylenglykolmonolaurat enthalten. Das mehrschichtige Laminat wird durch einen druckempfindlichen Haftkleber, der ebenfalls Resorptionsförderer enthält, fixiert. Der Nachteil dieser Formulierung ist:
- aufwendige technische Herstellung
- hohe Mengen an Resoptionsförderern können Hautirritationen auslösen
- dickes System und dadurch nicht hautanschmiegsam und variabler Kontakt, was zu unzuverlässigen Blutspiegeln führt.

US 4,911,916 beschreibt eine Diffusionsmatrix für die Anwendung von Fentanyl, bei der die Diffusionsmatrix aus einem wirkstoffgetränkten Polyurethanschaum besteht, der mit einem druckempfindlichen Silikonhaftkleber beschichtet ist.

US 5,719,197 beschreibt ein transdermales Matrixsystem, welches Wirkstoffe wie Fentanyl in Lösungen von Resorptionsförderern enthält, wobei die weichmachende Wirkung der Lösemittel durch Zusatz von Tonmineralien verringert werden muss. Nachteile:
- hohe hautirritierende Potenz durch grosse Mengen an Resorptionsförderern
- schwierige Verarbeitung der Masse durch Zusatz von Tonmineralien (Sedimentation)

Aus DE 196 54 468 ist ein transdermales therapeutisches System in Form eines Pflasters bekannt, das in seiner wirkstoffhaltigen haftklebenden Matrixschicht beispielsweise Fentanyl (Spalte 4 Zeile 21) und beispielsweise Aloe Vera Extrakt (Spalte 4 Zeile 67) enthalten kann. Fentanyl wird in einer großen Liste von Wirkstoffen und der Aloe Vera Extrakt in einer gleichfalls großen Liste pflanzlicher Zubereitungen genannt. Eine Kombination von Fentanyl und Aloe Vera Extrakt wird nicht angesprochen.

DE 197 08 674 beschreibt ein transdermales therapeutisches System mit einer wirkstoffhaltigen Trägerschicht (1), die als Wirkstoff beispielsweise Fentanyl (Spalte 6 Zeile 23) und als pflanzliche Zubereitung beispielsweise Aloe Vera Extrakt (Spalte 6 Zeilen 65/66) enthalten kann. Wiederum ist Fentayl in einer praktisch unbegrenzten Liste von Wirkstoffen und der Aloe Vera Extrakt in einer beträchtlichen Liste pflanzlicher Zubereitungen angeführt. Eine Kombination von Fentanyl und Aloe Vera Extrakt ist diesem Stand der Technik nicht zu entnehmen. Auch für diesen Stand der Technik ist zu betonen, daß er nicht ein Fentanyl-System vorsehen will, vielmehr ein transdermales therapeutisches System für eine praktisch unbegrenzte Zahl von Wirkstoffen, das sich dadurch auszeichnet, daß die wirkstoffhaltige Filmschicht (3) sandwich-artig zwischen einer Trennschicht (2) und einer Schutzschicht (4) vorgesehen ist, die beide von der wirkstoffhaltigen Filmschicht (3) entfernt werden können. Zur Entfernung der Schutzschicht (4) vergleiche Spalte 4 Zeilen 30/31 und zur Entfernung der Trennschicht vergleiche Spalte 4 Zeilen 36/42.

EP 1 170 004 beschreibt ein transdermales Matrixsystem, welches einen hydrierten Kolophoniumester als Kleberzusatz enthält. Die Permeation von Fentanyl wird erhöht durch die Zugabe von organischer Säure in der Matrix.

### 2. Aufgabe der Erfindung

Die Aufgabe der vorliegenden Erfindung ist es, ein Transdermalsystem mit
1. langanhaltender (3 bis 7 Tage) und kontinuierlicher Wirkstofffreisetzung von 5 bis 200 µg/h/System.
2. guter Hautverträglichkeit über die gesamte Tragedauer,
3. zuverlässiger Wirksamkeit,
4. Vermeidung missbräuchlicher Anwendung,
5. gutem Tragekomfort mit ausreichender Klebkraft,
6. preiswerter Herstellung bei gut reproduzierbarer Qualität,
7. Dosisanpassung durch einfache Flächenvariation,
8. hoher Lagerstabilität und
9. hoher mechanischer Stabilität durch homogenen Aufbau des Systems
zur Verfügung zu stellen.

### 3. Beschreibung der Erfindung

Die der Erfindung zugrundeliegende Aufgabe wird durch ein Transdermalsystem mit einem Gehalt an Fentanyl als Wirkstoff gelöst, wobei das Transdermalsystem aus
- einem Substrat,
- einer auf das Substrat aufgebrachten Mischung aus
   dem Wirkstoff
   einem öligen Aloe vera-Extrakt,
   einem Harz und
   einem Kleber sowie
- einer Schicht, die die auf dem Substrat aufgebrachten Mischung kaschiert, besteht oder die angeführten Komponenten umfaßt.

Das erfindungsgemäße Transdermalsystem kann durch ein Mazerat mit Sojaöl als Extraktionsmittel, insbesondere ein Mazerat aus Blättern der Aloe barbadensis, vorzugsweise aus frischen Blättern der Aloe barbadensis, als Aloe vera-Extrakt gekennzeichnet sein.

Ferner kann das erfindungsgemäße Transdermalsystem durch einen Aloe vera-Extrakt mit einem Gehalt von etwa 7 % Öl der Aloe-Blätter und etwa 93 % Sojaöl gekennzeichnet sein.

Ferner kann das erfindungsgemäße Transdermalsystem durch einen Ester von Kolophonium, einen hydrierten Kolophoniumester, ein synthetisches organisches Harz und/oder eine synthetische Kohlenwasserstoff-Verbindung als Harz gekennzeichnet sein.

Ferner kann das erfindungsgemäße Transdermalsystem durch ein Verhältnis von Aloe vera-Extrakt : Harz im Bereich von 1 : 10 bis 99 : 1, vorzugsweise 1 : 5 bis 50 : 1 und insbesondere 1 : 2 bis 15 : 1 gekennzeichnet sein (Gewichtsbasis).

Ferner kann das erfindungsgemäße Transdermalsystem durch ein thermoplastisches Elastomeres, insbesondere ein druckempfindliches thermoplastisches Elastomeres, als Kleber bzw. Haftkleber gekennzeichnet sein, insbesondere durch ein thermoplastisches Elastomeres auf Basis eines Block-Copolymeren, vorzugsweise eines Styrol-Butadien-Styrol-Block-Copolymeren (SBS), eines Styrol-Isopren-Styrol-Block-Copolymeren (SIS) oder eines Styrol-Ethylen/Butadien-Styrol-Block-Copolymeren (SE/BS), oder einen Kohlenwasserstoffkleber, vorzugsweise einen Acrylat- oder einen Polyisobutylen-Kleber, oder einen Silikonkleber.

Ferner kann das erfindungsgemäße Transdermalsystem dadurch gekennzeichnet sein, dass es außer den vorstehend angeführten Komponenten keine zusätzliche Fixierhilfe aufweist.

Ferner kann das erfindungsgemäße Transdermalsystem durch ein Verhältnis von Fentanyl : Kleber im Bereich von 0.01 : 1 bis 1 : 1 und vorzugsweise 0.1 : 1 gekennzeichnet sein.

Ferner kann das erfindungsgemäße Transdermalsystem durch eine silikonisierte Kunststoffolie, insbesondere eine silikonisierte Polyesterfolie, eine fluorierte Kunstoffolie, oder silikonisiertes Papier als Substrat gekennzeichnet sein.

Ferner kann das erfindungsgemäße Transdermalsystem durch eine Kunststoffolie, insbesondere eine Polyesterfolie, ein Vlies, einen Kunststoffschaum oder ein Gewebe als kaschierende Schicht gekennzeichnet sein.

Schließlich kann das erfindungsgemäße Transdermalsystem durch ein Gewicht/Flächeneinheit im Bereich von 20 bis 200 g/m² gekennzeichnet sein.

### 4. Detaillierte Beschreibung der Erfindung

Bei den Entwicklungsarbeiten wurde gefunden, dass eine Mischung aus Fentanyl, einem öligen Aloe vera-Extrakt, einem Harz und einem Kleber unabhängig von seinen chemischen Eigenschaften, nämlich bestehend aus thermoplastischen Elastomeren basierend auf Block-Copolymeren, z.B. Styrol-Butadien-Styrol (SBS), Styrol-Isopren-Styrol (SIS) oder Styrol-Ethylen/Butadien-Styrol (SE/BS), oder auf Acrylat- oder Polyisobutylenklebern [Kohlenwasserstoffkleber] oder auf Silikonklebern, alle obengenannte Ziele erreicht, d.h. es wird ein über mindestens 3 Tage konstanter Flux aufrechterhalten, während gleichzeitig eine optimale Klebkraft beobachtet wird. Es handelt sich um ein Transdermalsystem, das zudem sehr dünn, nämlich zwischen 20 und 200 g/m² ist, und das sich durch seine Flexibilität sehr gut an die jeweiligen Tragestellen anschmiegen kann. Dadurch wird gewährleistet, dass die konstante Abgaberate erhalten bleibt. Überraschenderweise wurde gefunden, dass die Wirkstoffabgabekontrolle durch Variation des Konzentrationsverhältnisses zwischen Aloe vera-Extrakt und Harz ermöglicht wird. Dadurch kann ein Fentanyl-Flux durch die Haut eingestellt werden, der den therapeutischen Effekt gewährleisten kann. Das Verhältnis zwischen Aloe vera-Extrakt und Harz soll maximal 0,1 bis 99 sein, im besonderen z.B. 0,5 bis 15.

Fentanyl-Base ist in ausreichender Konzentration in dem Klebstoffpolymer löslich in einem Verhältnis Fentanyl : Kleber von maximal 0,01 bis 1 : 1, im speziellen z.B. 0,1 : 1, und das chemische Potential von Fentanyl in dem Polymer ist auch ohne Zumischung weiterer Komponenten hoch genug, um über mindestens 3 Tage einen ausreichenden Wirkstoff-Flux durch intakte Haut aufrecht zu erhalten .

**Vergleichsbeispiel 1** gibt die in vitro-Hautpermeation des handelsüblichen Reservoirsystems Durogesic^{®} TTS wieder, bei dem die Wirkstoffpermeation mittels des Resorptionsförderers Ethanol beeinflusst wird. Das **Vergleichsbeispiel 2** gibt die in vitro-Hautpermeation einer Fentanyl-Matrix, bestehend aus einem Styrol-Isopren-Styrol-Kleber (SIS) ohne weitere Zusätze wieder. Die Hautpermeation ist vergleichbar der des **Vergleichsbeispiels 1.** Der Nachteil des Systems gemäß **Vergleichsbeispiel 2** ist die nicht ausreichende Klebekraft, um eine für 3 oder 7 Tage zuverlässige Wirksamkeit zu gewährleisten.

Die Klebkraft des Transdermalsystems entsprechend vorliegender Erfindung wird nun durch Harzzusatz so hoch, daß eine ausgezeichnete Klebkraft über drei bis sieben Tage ohne Hautirritationen erreicht wird. Von der Verwendung zusätzlicher Fixierhilfen kann abgesehen werden. Während Harze, z.B. Foral E105 (ein Ester des Kolophoniums), die Freisetzung erniedrigen, wirkt der ölige Aloe vera-Extrakt freisetzungsfördernd (s. **Beispiel 1**). Man kann also durch Wahl des geeigneten Aloe-Extrakt/Harz-Verhältnisses die Freisetzung des Fentanyls aus den erfindungsgemässen Transdermalsystemen auf das therapeutisch geforderte Niveau einstellen und dabei eine ausgezeichnete Klebkraft erhalten.

Aloe vera-Extrakt ist ein Mazerat aus frischen Blättern der Aloe vera (L.) bzw. der Aloe barbadensis (Mill.) mit Sojaöl als Extraktionsmittel. Der Extrakt enthält 7 % Öl der Aloe-Blätter und 93 % Sojaöl. Harze sind Ester des Kolophoniums oder hydrierte Kolophoniumester oder synthetische KohlenwasserstoffVerbindungen.

Die Herstellung von Fentanylpflastern kann auf herkömmlichen Maschinen, die dem Fachmann bekannt sind, vorgenommen werden: Fentanyl-Base wird in einem geeigneten, leicht flüchtigen Lösemittel, z.B. Methanol, Ethanol, Isopropanol, Dioxan oder n-Heptan gelöst oder dispergiert. Die Lösung/Dispersion wird mit einer Lösung des oben beschriebenen druckempfindlichen Haftklebers unter Zusatz von Aloe vera-Extrakt und Harz in einem geeigneten Gefäß gemischt, ggfs. (aber nicht zwingend notwendig) können übliche Stoffe wie Füllmittel, Hautschutzstoffe, Klebrigmacher o.ä. zugesetzt werden. Die Mischung des Fentanyls mit dem Klebstoff und ggfs. weiteren Stoffen kann in einer üblichen Beschichtungsmaschine auf ein Substrat, z.B. silikonisierte oder fluorierte Kunststoffolie, silikonisiertes Papier o.ä., üblicherweise als eine Schicht aufgetragen und in einem nachfolgenden Trockner vom Lösemittel befreit werden. In besonderen Fällen können aber auch mehrere Schichten der gleichen oder unterschiedlicher Zusammensetzung aufgetragen werden. Nach Verlassen des Trockners kann die nun getrocknete und selbstklebende Wirkstoff/Klebstoffmatrix mit einer weiteren Schicht kaschiert werden, die z.B. eine Kunststoffolie, ein Vlies, ein Kunststoffschaum, ein Gewebe o.ä sein kann.

In einem weiteren Verarbeitungsschritt können in einer dem Fachmann bekannten Schneide- oder Stanzvorrichtung die gewünschten Transdermalsysteme mit definierter Form und Größe ausgeschnitten oder gestanzt werden. Die fertigen Systeme können zum Schutz in Beutel oder ähnliche Verpackungen eingebracht werden. Typischerweise enthält die Matrix der Systeme Fentanyl im Konzentrationsbereich zwischen 0,1 und 20 %, bevorzugt zwischen 2 und 10 %. Das Flächengewicht der getrockneten Matrix liegt üblicherweise zwischen 20 und 200 g/m², bevorzugt zwischen 50 und 120 g/m². Die Abgaberate liegt im Bereich zwischen 5 bis 200 µg Fentanyl/h/System, bevorzugt zwischen 10 und 100 µg/h/System.

Zur Charakterisierung der Transdermalsysteme im Hinblick auf ihre Wirkstoffabgabe werden im wesentlichen zwei Methoden verwendet:
1. in vitro-Hautpermeationsuntersuchungen
2. in vitro-Freisetzungsuntersuchungen nach gültigen Pharmakopöen

Hautpermeationsuntersuchungen werden häufig an isolierter Haut von Nacktmäusen durchgeführt. Dabei wird ein Pflasterstück auf die Oberseite der Haut geklebt und dieser Verbund in einer Diffusionszelle montiert. Eine Pufferlösung (Akzeptor) tritt dabei mit der Unterseite der Haut in Kontakt, und es wird die zeitabhängige Konzentrationsänderung im Akzeptormedium z.B. mittels einer hochdruckflüssigchromatografischen Analysenmethode gemessen. Die Ergebnisse, die mit den erfindungsgemäßen Zubereitungen erhalten werden, sind in den folgenden Beispielen aufgeführt.

Die in vitro-Freisetzungsuntersuchungen werden in Glasgefäßen ausgeführt, die nach den Bestimmungen der Pharmakopöen aufgebaut sind. In einem zylindrischen 1-Liter-Gefäß mit rundem Boden wird das Pflaster auf einer Siebplatte so befestigt, daß die Klebeschicht nach oben weist. Die Siebplatte wird auf den Boden des Gefäßes gebracht, das Gefäß mit Wasser gefüllt und mit einem definierten Rührer zum Konzentrationsausgleich gerührt. Hierbei wird ebenfalls die zeitabhängige Konzentration im Freisetzungsmedium gemessen. Die Ergebnisse dieser Untersuchungen sind in den Beispielen aufgeführt. Der Unterschied der Methoden besteht darin, daß die Freisetzungsuntersuchungen das Freisetzungsverhalten des Wirkstoffes aus dem Pflaster beschreiben, was jedoch in der Regel nicht mit der biologischen Wirkung korreliert. Das Hautpermeationsmodell beinhaltet dagegen zusätzlich zur notwendigen Freisetzung den Verteilungsschritt des Wirkstoffes in die Haut und die Diffusion durch die Haut. Hiermit sind in der Regel Korrelationen zur biologischen Wirkung möglich.

### 5. Beispiele

### Vergleichsbeispiel 1

Ein handelsübliches Fentanyl-Pflaster, Durogesic^{®} TTS, mit folgender Charakteristik (entsprechend US Patent 4,588,580):

| | |
|---|---|
| Gehalt Fentanyl: | 2,5 mg |
| Fläche: | 10 cm² |

### Zusammensetzung (qualitativ) :

Silikonkleber
Ethanol/Wasser
Hydroxyethylcellulose
mikroporöse Ethylenvinylacetat-Membran (EVA)
wurde einer in vitro-Hautpermeationsuntersuchung in einem Mäusehautmodell unterworfen. Das Ergebnis ist in Tabelle 1 dargestellt.

### Hautpermeation

Ein 1,5 cm² großes Stück Haut von weiblichen Nacktmäusen, das von Unterhautgewebe befreit wurde, wird auf die genau 1 cm² große Öffnung einer automatisierten Diffusionszelle gelegt, mit einem ca 1,5 cm² großen Stück des Fentanyl-Pflasters beklebt und mit einer Andruckvorrichtung auf der Zelle abgedichtet. Die Zelle wird mit 15 ml einer physiologischen HEPES-Pufferlösung gefüllt und auf 32 °C temperiert. Nach definierten Zeiten werden aus der Pufferlösung Proben gezogen und der Wirkstoffgehalt in ihnen mittels hochdruckflüssigchromatografischem Analyseverfahren (HPLC) ermittelt. Nach diesem Verfahren werden alle unten beschriebenen Pflaster untersucht. Die Ergebnisse sind in Tabelle 1 dargestellt.

### Vergleichsbeispiel 2

Als Vergleich wurde ein Matrix-Fentanylpflaster unter Verwendung eines SIS-Klebers ohne Hinzufügen von Harz und Aloe vera-Öl hergestellt. Das System hatte folgende Charakteristik:

| | |
|---|---|
| Gehalt Fentanyl: | 4,5 mg |
| Fläche: | 10 cm² |

### Zusammensetzung

| | |
|---|---|
| SIS (Styrol-Isopren-Styrol) Duro-Tak* 387-6173: | 85,5 mg |
| silikonisierte Polyesterfole FL200075 1S**: | 10 cm² |
| Polyester Folie Hostaphan MN 19 MED***: | 10 cm² |

| | |
|---|---|
| * National Starch & Chemical, NL-Zutphen ** Loparex, NL-Apeldoorn *** Mitsubishi Polyester Foils, D-Frankfurt | |

### Herstellung

Fentanyl wird in Ethanol gelöst. Die Lösung wird einer ausreichenden Menge der handelsüblichen Klebstofflösung zugegeben und mittels eines Rührers homogenisiert. Die homogene Lösung wird mittels eines Ziehrakels auf einen Bogen einer silikonisierten Polyesterfolie (ca 75 µm) in definierter Schichtdicke ausgestrichen. Der Bogen wird anschließend zur Trocknung 30 min bei 50 °C in einem Trockenschrank getrocknet. Danach wird eine ca 19 µm dicke Polyesterfolie auf die klebende Schicht aufkaschiert. Aus dem fertigen Laminat werden mittels einer Handstanze 10 cm² große Pflaster ausgestanzt.

### Hautpermeation

### s. Vergleichsbeispiel 1. Die Ergebnisse sind in Tabelle 1 dargestellt.

### Vergleichsbeispiel 3

Als Vergleich wurde ein Fentanylpflaster unter Verwendung eines SIS-Klebers mit Hinzufügen von Aloe vera-Öl hergestellt. Das System hatte folgende Charakteristik:

| | |
|---|---|
| Gehalt Fentanyl: | 4,5 mg |
| Fläche: | 10 cm² |

### Zusammensetzung

| | |
|---|---|
| SIS (Styrol-Isopren-Styrol) Duro-Tak* 387-6173 | 81 mg |
| Aloe vera-Öl | 4,5 mg |
| silikonisierte Polyesterfolie FL200075 1S** | 10 cm² |
| Polyester Folie Hostaphan MN 19 MED*** | 10 cm² |

| | |
|---|---|
| * National Starch & Chemical, NL-Zutphen ** Loparex, NL-Apeldoorn *** Mitsubishi Polyester Foils, D-Frankfurt | |

### Herstellung

### s. Vergleichsbeispiel 2

### Hautpermeation

### s. Vergleichsbeispiel 1. Die Ergebnisse sind in Tabelle 1 dargestellt.

### Vergleichsbeispiel 4

Als Vergleich wurde ein Fentanylpflaster unter Verwendung eines SIS-Klebers mit Hinzufügen von Harzen hergestellt. Das System hatte folgende Charakteristik:

| | |
|---|---|
| Gehalt Fentanyl: | 4,5 mg |
| Fläche: | 10 cm² |

### Zusammensetzung

| | |
|---|---|
| SIS (Styrol-Isopren-Styrol) Duro-Tak 387-6173: | 76,5 mg |
| Harz (Foral 105E) | 9 mg |
| silikonisierte Polyesterfolie FL200075 1S** | 10 cm² |
| Polyester-Folie Hostaphan MN 19 MED*** | 10 cm² |

### Herstellung

### s. Vergleichsbeispiel 2

### Hautpermeation

s. **Vergleichsbeispiel 1.** Die Ergebnisse sind in Tabelle 1 dargestellt.

**Tabelle 1 In vitro-Hautpermeation von Fentanyl-Matrixpflastern**

| ***Zeit(Stunden)*** | ***Vergl. Beispiel 1*** | ***Vergl. Beispiel 2*** | ***Vergl. Beispiel 3*** | ***Vergl. Beispiel 4*** | ***Beispiel*** |
|---|---|---|---|---|---|
| | Permeation Fentanyl (µg/cm²) | | | | |
| 6 | 26,18 | 24,2 | 22,4 | 14,6 | 35,9 |
| 15 | 62 | 67,2 | 83,7 | 46,9 | 88,4 |
| 24 | 99,8 | 109,3 | 147,9 | 81,1 | 133,8 |
| 36 | 147,4 | 160 | 227,1 | 123,2 | 178,9 |
| 48 | 190,4 | 204,6 | 298,6 | 160 | 214,7 |
| 60 | 226,8 | 240,3 | 353,2 | 188,6 | 240,3 |

### Beispiel 1

Ein erfindungsgemäßes Fentanylpflaster unter Verwendung unter der Kombination von Aloe vera Extrakt und einem Harz hat folgende Charakteristik:

| | |
|---|---|
| Gehalt Fentanyl: | 5 mg |
| Fläche: | 10 cm² |

### Zusammensetzung

| | |
|---|---|
| Polyisobutylen PSA (MA24A): | 61,7 mg |
| Aloe vera Extrakt | 7,5 mg |
| Harz (Foral 105E) | 0,75 mg |
| silikonisierte Polyesterfolie FL200075 1S** | 10 cm² |
| Polyester-Folie Hostaphan MN 19 MED*** | 10 cm² |

### Herstellung

### s. Vergleichsbeispiel 2

### Hautpermeation

s. **Vergleichsbeispiel 1.** Die Ergebnisse sind in Tabelle 1 dargestellt.

### 6. Diskussion

Das **Vergleichsbeispiel 1** in der Tabelle 1 zeigt die Resultate einer Hautpermeation, die mit dem Vergleichpräparat Durogesic^{®} TTS erhalten wurde, mit 227 µg Fentanyl/cm² bei 60 Stunden. Das **Vergleichsbeispiel 2** zeigt mit 240 µg/cm² bei 60 Stunden die Resultate eines einfachen Matrixsystems mit dem beschriebenen Kleber ohne zusätzliche Hilfsstoffe; es zeigt ferner als Basisrezeptur eine vergleichbare, etwas höhere Permeation nach 3 Tagen als **Vergleichsbeispiel 1.** Diese Rezeptur ist jedoch nicht für eine mehrtägige Applikation geeignet, da die Klebkraft nicht ausreichend hoch ist. **Vergleichsbeispiel 4** zeigt daher die Hautpermeation einer Rezeptur mit sehr guten Klebeeigenschaften, erhalten durch Zusatz von Kolophoniumharz. Dabei sinkt jedoch die Permeation des Fentanyls unter die des **Vergleichsbeispiels 1.** Als **Vergleichsbeispiel 3** ist eine Rezeptur aufgeführt, die als freisetzungsfördernde Substanz öligen Aloe vera-Extrakt enthält, der die permeierte Menge an Fentanyl um annähernd 50 % gegenüber **Vergleichsbeispiel 2** steigert. Um für eine Therapie optimale Fentanylpermeation zu erreichen, wird dieser Rezeptur z.B. ein Harz aus dem **Vergleichsbeispiel 4** zugesetzt (Foral E 105), damit auch eine ausreichende Klebkraft über 3 bis 7 Tage gewährleistet ist (siehe **Beispiel 1**), wobei die permeierten Mengen auf annähernd die Werte des **Vergleichsbeispiels 1** sinken, die jedoch ausreichend für einen gewünschten therapeutischen Effekt sind.

## Patentansprüche

1. Transdermalsystem mit einem Gehalt an Fentanyl als Wirkstoff und bestehend aus
- einem Substrat,
- einer auf das Substrat aufgebrachte Mischung aus
dem Wirkstoff,
einem öligen Aloe vera-Extrakt,
einem Harz und
einem Kleber sowie
- einer Schicht, die die auf dem Substrat aufgebrachte Mischung kaschiert.

2. Transdermalsystem nach Anspruch 1, **gekennzeichnet durch** ein Mazerat mit Sojaöl als Extraktionsmittel, insbesondere ein Mazerat aus Blättern der Aloe barbadensis, vorzugsweise aus frischen Blättern der Aloe barbadensis, als Aloe vera-Extrakt.

3. Transdermalsystem nach Anspruch 1 oder 2, **gekennzeichnet durch** eine Fentanyl-Konzentration von 0,1 bis 20 % und insbesondere 2 bis 10 % in der Mischung mit Aloe vera-Extrakt, Harz und Kleber.

4. Transdermalsystem nach einem der vorhergehenden Ansprüche, **gekennzeichnet durch** einen Aloe vera-Extrakt mit einem Gehalt von 7 % Öl der Aloe-Bätter und 93 % Sojaöl (Gewichtsbasis).

5. Transdermalsystem, nach einem der vorhergehenden Ansprüche, **gekennzeichnet durch** einen Ester von Kolophonium, einen hydrierten Kolophoniumester, ein synthetisches organisches Harz und/oder eine synthetische Kohlenwasserstoff-Verbindung als Harz.

6. Transdermalsystem nach einem der vorhergehenden Ansprüche, **gekennzeichnet durch** ein Verhältnis von Aloe vera-Extrakt : Harz im Bereich von 0,1 : 1 bis 99 : 1, vorzugsweise 0,2 : 1 bis 50 : 1 und insbesondere 0,5 : 1 bis 15 : 1 (Gewichtsbasis).

7. Transdermalsystem nach einem der vorhergehenden Ansprüche, **gekennzeichnet durch** ein thermoplastisches Elastomeres, insbesondere ein druckempfindliches thermoplastisches Elastomeres, als Kleber, insbesondere ein thermoplastisches Elastomeres auf Basis eines Block-Copolymeren, vorzugsweise eines Styrol-Butadien-Styrol-Block-Copolymeren (SBS), eines Styrol-Isopron-Styrol-Block-Copolymeren (SIS) oder eines Styrol-Ethylen/Butadien-Styrol-Block-Copolymeren (SE/BS), oder einen Kohlenwasserstoffkleber, vorzugsweise einen Acrylat- oder einen Polyisobutylen-Kleber, oder einen Silikonkleber.

8. Transdermalsystem nach einem der vorhergehenden Ansprüche, **dadurch gekennzeichnet, daß** das Transdermalsystem außer den Komponenten gemäß einem der vorhergehenden Ansprüche keine zusätzliche Fixierhilfe aufweist.

9. Transdermalsystem nach einem der vorhergehenden Ansprüche, **gekennzeichnet durch** ein Verhältnis Fentanyl : Kleber im Bereich von 0.01 : 1 bis 1 : 1 und vorzugsweise 0.1 : 1 (Gewichtsbasis).

10. Transdermalsystem nach einem der vorhergehenden Ansprüche, **gekennzeichnet durch** eine abziehbare Schutzschicht, vorzugsweise eine silikonisierte Kunststoffolie, insbesondere eine silikonisierte Polyesterfolie, eine fluorierte Kunststoffolie oder silikonisiertes Papier als Substrat.

11. Transdermalsystem nach einem der vorhergehenden Ansprüche, **gekennzeichnet durch** eine Kunststoffolie, insbesondere eine Polyesterfolie, ein Vlies, einen Kunststoffschaum oder ein Gewebe als kaschierende Schicht.

12. Transdermalsystem nach einem der vorhergehenden Ansprüche, **dadurch gekennzeichnet, daß** die kaschierende Schicht als wirkstoffundurchlässige Deckschicht ausgebildet ist.

13. Transdermalystem nach einem der vorhergehenden Ansprüche, **gekennzeichnet durch** ein Gewicht/Flächeneinheit der Matrix im Bereich von 20 bis 200 g/m² und vorzugsweise 50 bis 120 g/m².

14. Transdermalsystem nach einem der vorhergehenden Ansprüche, **gekennzeichnet durch** einen Gehalt an einem üblichen Füllmittel, Hautschutzstoff und/oder Klebrigmacher.

15. Transdermalsystem nach einem der vorhergehenden Ansprüche, **dadurch gekennzeichnet, dass** das System in einer Verpackung oder einem Beutel vorgesehen ist.

## Claims

1. A transdermal system containing fentanyl as the active ingredient and consisting of
- a substrate,
- a mixture of the following ingredients applied to the substrate,
the active ingredient,
an oily aloe vera extract,
a resin, and
an adhesive, as well as
- a layer laminated to the mixture applied to the substrate.

2. The transdermal system according to Claim 1, **characterized by** a macerate containing soya oil as extraction agent, in particular a macerate of leaves of *Aloe barbadensis,* preferably of fresh leaves of *Aloe barbadensis*, as aloe vera extract.

3. The transdermal system according to Claim 1 or 2, **characterized by** a fentanyl concentration of 0.1 to 20 %, and in particular 2 % to 10 %, in the mixture with aloe vera extract, resin and adhesive.

4. The transdermal system according to one of the preceding claims, **characterized by** an aloe vera extract containing 7 % oil of aloe leaves and 93 % soya oil (based on weight).

5. The transdermal system according to one of the preceding claims, **characterized by** an ester of colophonium, a hydrogenated colophonium ester, a synthetic organic resin and/or a synthetic hydrocarbon compound as the resin.

6. The transdermal system according to one of the preceding claims, **characterized by** a ratio of aloe vera extract to resin in the range of 0.1:1 to 99:1, preferably 0.2:1 to 50:1 and in particular 0.5:1 to 15:1 (based on weight).

7. The transdermal system according to one of the preceding claims, **characterized by** a thermoplastic elastomer, in particular a pressure-sensitive thermoplastic elastomer, as the adhesive, in particular a thermoplastic elastomer based on a block copolymer, preferably a styrenebutadiene-styrene block copolymer (SBS), a styreneisoprene-styrene block copolymer (SIS) or a styrene-ethylene/butadiene-styrene block copolymer (SE/BS), or a hydrocarbon adhesive, preferably an acrylate adhesive or a polyisobutylene adhesive, or a silicone adhesive.

8. The transdermal system according to one of the preceding claims, **characterized in that** the transdermal system does not have any additional fixative aid except for the components according to one of the preceding claims.

9. The transdermal system according to one of the preceding claims, **characterized by** a fentanyl-to-adhesive ratio in the range of 0.01:1 1 to 1:1, and preferably 0.1:1 (based on weight).

10. The transdermal system according to one of the preceding claims, **characterized by** a removable protective layer, preferably a siliconized plastic film, in particular a siliconized polyester film, a fluorinated plastic film or siliconized paper as substrate.

11. The transdermal system according to one of the preceding claims, **characterized by** a plastic film, in particular a polyester film, a nonwoven web, a plastic foam or a fabric as laminating layer.

12. The transdermal system according to one of the preceding claims, **characterized in that** the laminating layer is designed as a covering layer, which is impermeable for the active ingredient.

13. The transdermal system according to one of the preceding claims, **characterized by** a ratio of the weight per unit of area of the matrix in the range of 20 to 200 g/m², preferably 50 to 120 g/m².

14. The transdermal system according to one of the preceding claims, **characterized by** the fact that it contains a conventional filler, a skin-protective agent and/or tackifying agent.

15. The transdermal system according to one of the preceding claims, **characterized in that** the system is provided in a package or a bag.

## Revendications

1. Système transdermique avec une teneur en fentanyl comme principe actif et consistant en
- un substrat,
- un mélange appliqué sur le substrat,
du principe actif,
d'un extrait d'aloe vera huileux,
d'une résine et
d'un agent adhésif, ainsi que
- une couche qui est contrecollée sur le mélange appliqué sur le substrat.

2. Système transdermique selon la revendication 1, **caractérisé par** un macérat avec de l'huile de soya comme agent d'extraction, en particulier un macérat de feuilles de l'Aloe barbadensis, de préférence de feuilles fraîches de l'Aloe barbadensis, comme extrait d'aloe vera.

3. Système transdermique selon la revendication 1 ou 2, **caractérisé par** une concentration en fentanyl de 0,1 à 20 % et en particulier de 2 à 10 % dans le mélange avec l'extrait d'aloe vera, la résine et l'agent adhésif.

4. Système transdermique selon l'une quelconque des revendications précédentes, **caractérisé par** un extrait d'aloe vera d'une teneur en huile des feuilles d'aloe de 7 % et en huile de soya de 93 % (base pondérale).

5. Système transdermique selon l'une quelconque des revendications précédentes, **caractérisé par** un ester de colophane, un ester de colophane hydrogéné, une résine organique synthétique et/ou un composé hydrocarbure synthétique comme résine.

6. Système transdermique selon l'une des revendications précédentes, **caractérisé par** un rapport de l'extrait d'aloe vera à la résine allant de 0,1:1 à 99:1, de préférence de 0,2:1 à 50:1 et particulièrement de 0,5:1 à 15:1 (base pondérale).

7. Système transdermique selon l'une quelconque des revendications précédentes, **caractérisé par** un élastomère thermoplastique, en particulier un élastomère thermoplastique sensible à la pression, comme agent adhésif, en particulier un élastomère thermoplastique sur la base d'un copolymère séquencé, de préférence d'un copolymère séquencé styrène-butadiène-styrène (SBS), d'un copolymère séquencé styrène-isoprène-styrène (SIS) ou d'un copolymère séquencé styrène-éthylène/butadiène-styrène (SE/BS), ou un agent adhésif hydrocarbure, de préférence un agent adhésif acrylique ou un agent adhésif polyisobutylène, ou un agent adhésif silicone.

8. Système transdermique selon l'une des revendications précédentes, **caractérisé en ce que** le système transdermique ne présente pas d'aide de fixation supplémentaire en dehors des composants selon l'une quelconque des revendications précédentes.

9. Système transdermique selon l'une quelconque des revendications précédentes, **caractérisé par** un rapport fentanyl:agent adhésif allant de 0,01:1 à 1:1 et de préférence de 0,1:1 (base pondérale).

10. Système transdermique selon l'une quelconque des revendications précédentes, **caractérisé par** une couche de protection retirable, de préférence une feuille de matière plastique siliconée, en particulier une feuille de polyester siliconée, une feuille de matière plastique fluorée ou un papier siliconé comme substrat.

11. Système transdermique selon l'une quelconque des revendications précédentes, **caractérisé par** une feuille de matière plastique, en particulier une feuille de polyester, un non-tissé, une mousse de matière plastique ou un tissu comme couche de contrecollage.

12. Système transdermique selon l'une quelconque des revendications précédentes, **caractérisé en ce que** la couche de contrecollage est réalisée comme une couche de revêtement imperméable au principe actif.

13. Système transdermique selon l'une quelconque des revendications précédentes, **caractérisé par** un rapport poids/unité de plan de la matrice allant de 20 à 200 g/m² et de préférence de 50 à 120 g/m².

14. Système transdermique selon l'une quelconque des revendications précédentes, **caractérisé par** une teneur en une matière de remplissage, une substance de protection pour la peau et/ou un agent poisseux traditionnels.

15. Système transdermique selon l'une quelconque des revendications précédentes, **caractérisé en ce que** le système est prévu dans un emballage ou dans un sachet.
